# DEMANDE DE BREVET EUROPEEN

(11) **EP 1 043 014 A1**
(43) Date de publication de la demande: **11.10.2000**
(21) Numéro de dépôt: 00400296.0
(22) Date de dépôt: 03.02.2000
(51) Int. Cl.: A61K 7/42

(54) **Compositions cosmétiques antisolaires à base d'un mélange synergique de filtres et utilisations**

(30) Priorité: 12.02.1999 FR 9901733
(71) Demandeur: L'OREAL, 75008 Paris (FR)
(72) Inventeur: Candau, Didier, 91570 Bievres (FR)
(74) Mandataire: Miszputen, Laurent

(57) **Abrégé**

L'invention concerne de nouvelles compositions cosmétiques à usage topique, en particulier pour la photoprotection de la peau et/ou des cheveux, caractérisées par le fait qu'elles comprennent, dans un support cosmétiquement acceptable notamment de type huile-dans-eau, (a) de l'acide benzène 1 ,4-di(3-méthylidène-10-camphosulfonique), éventuellement sous forme partiellement ou totalement neutralisée, à titre de premier filtre, (b) au moins un dérivé bis-résorcinyl triazine à titre de deuxième filtre, et (c), à titre de troisième filtre, au moins un composé comportant au moins deux groupes benzoazolyle ou un composé comportant au moins un groupe benzodiazolyle; lesdits premier, second et troisième filtres étant présents dans lesdites compositions dans une proportion produisant une activité synergique au niveau des indices de protection conférés.

L'invention concerne également leur application à la protection de la peau et des cheveux contre les effets du rayonnement ultraviolet.

## Description

La présente invention concerne de nouvelles compositions cosmétiques à usage topique plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires comprenant, dans un support cosmétiquement acceptable, une association entre (a) de l'acide benzène 1,4-di(3-méthylidène-10-camphosulfonique), éventuellement sous forme partiellement ou totalement neutralisée, à titre de premier filtre, (b) au moins un dérivé bis-résorcinyl triazine à titre de deuxième filtre, et (c), à titre de troisième filtre, au moins un composé comportant au moins deux groupes benzoazolyle ou un composé comportant au moins un groupe benzodiazolyle ; lesdits premier, second et troisième filtres étant présents dans lesdites compositions dans une proportion produisant une activité synergique au niveau des indices de protection conférés.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est-à-dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction de l'indice de protection recherché (l'indice de protection (IP) s'exprimant mathématiquement par le rapport du temps d'irradiation nécessaire pour atteindre le seuil érythématogène avec le filtre UV au temps nécessaire pour atteindre le seuil érythématogène sans filtre UV).

Or, à la suite d'importantes recherches menées dans le domaine de la photoprotection évoqué ci-dessus, la Demanderesse a découvert, de façon inattendue et surprenante, que la combinaison de trois filtres solaires particuliers et déjà connus en soi dans l'état de l'art, permettait, du fait d'un effet de synergie remarquable, d'obtenir des compositions antisolaires présentant des indices de protection nettement améliorés.

Cette découverte est à la base de la présente invention.

Ainsi, conformément à l'un des objets de la présente invention, il est maintenant proposé de nouvelles compositions cosmétiques, en particulier antisolaires, qui sont essentiellement caractérisées par le fait qu'elles comprennent, dans un support cosmétiquement acceptable, (a) de l'acide benzène 1,4-di(3-méthylidène-10-camphosulfonique), éventuellement sous forme partiellement ou totalement neutralisée, à titre de premier filtre, (b) au moins un dérivé bis-résorcinyl triazine à titre de deuxième filtre et (c) à titre de troisième filtre, au moins un compose comportant au moins deux groupes benzoazolyle ou un composé comportant au moins un groupe benzodiazolyle ; lesdits premier, second et troisième filtres étant présents dans lesdites compositions dans une proportion produisant une activité synergique au niveau des indices de protection conférés.

La présente invention a également pour objet l'utilisation de telles compositions pour la fabrication de, compositions cosmétiques destinées à la protection de la peau et/ou des cheveux contre le rayonnement ultraviolet, en particulier le rayonnement solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.

D'autres caractéristiques, aspects et avantages de la présente invention apparaîtront à la lecture de la description détaillée qui va suivre.

Ainsi, conformément à l'un des objets de la présente invention, il est maintenant proposé de nouvelles compositions cosmétiques ou dermatologiques, en particulier antisolaires, qui sont essentiellement caractérisées par le fait qu'elles comprennent, dans un support cosmétiquement acceptable:
(a) à titre de premier filtre, de l'acide benzène 1,4-di(3-méthylidène-10-camphosulfonique), éventuellement sous forme partiellement ou totalement neutralisée ;
(b) à titre de deuxième filtre, au moins un dérivé de bis-résorcinyl triazine ;
(c) à titre de troisième filtre, au moins un composé comportant au moins deux groupes benzazolyle par molécule ou bien un composé comportant, par molécule, au moins un groupe benzodiazolyle ; lesdits premier, second et troisième filtres étant présents dans une proportion produisant une activité synergique au niveau des indices de protection solaire conférés.

On entendra dans la description par 〈〈comportant au moins deux groupes benzoazolyle〉〉 comme comportant par molécule au moins deux groupes du type benzoxazolyle, benzothiazolyle ou benzimidazolyle.

On entendra dans la description par 〈〈comportant au moins un groupe benzodiazolyle〉〉 comme comportant par molécule un groupe du type benzodioxazolyle, benzodithiazolyle ou benzodiimidazolyle

L'acide benzène 1,4-di(3-méthylidène-10-camphosulfonique) et ses différents sels, décrits notamment dans les demandes de brevets FR-A-2528420 et FR-A-2639347, sont des filtres déjà connus en soi (filtres dits à large bande) capables en effet d'absorber les rayons ultraviolets de longueur d'ondes comprises entre 280 et 400 nm, avec des maxima d'absorption compris entre 320 et 400 nm, en particulier auxalentours de 345 nm.

Ces filtres répondent à la formule générale (I) suivante : 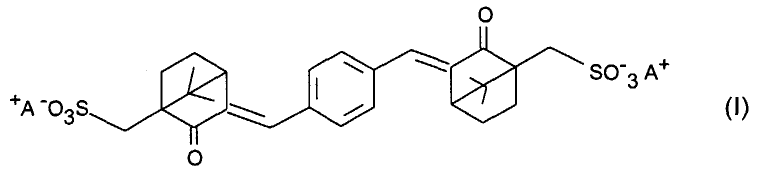 dans laquelle A désigne un atome d'hydrogène, un métal alcalin ou encore un radical NH(R)₃⁺ dans lequel les radicaux R, qui peuvent être identiques ou différents, désignent un atome d'hydrogène, un radical alkyle ou hydroxyalkyle en C₁-C₄ ou encore un groupement Mⁿ⁺/n, Mⁿ⁺ désignant un cation métallique polyvalent dans lequel n est égal à 2 ou 3 ou 4, Mⁿ⁺ désignant de préférence un cation métallique choisi parmi Ca²⁺, Zn²⁺, Mg²⁺, Ba²⁺, Al³⁺ et Zr⁴⁺. Il est bien entendu que les composés de formule (I) ci-dessus peuvent donner lieu à l'isomère "cis-trans" autour d'une ou plusieurs double(s) liaison(s) et que tous les isomères rentrent dans le cadre de la présente invention.

L'acide benzène 1,4-di(3-méthylidène-10-camphosulfonique) et ou l'un de ses différents sels est généralement présent dans les compositions filtrantes selon l'invention à une concentration totale allant de 0,1 à 15 % en poids environ, et de préférence de 0,2 à 10 % en poids environ, par rapport au poids total de la composition.

Les dérivés de bis-résorcinyl triazine, conformes à la présente invention sont choisis de préférence parmi ceux répondant à la formule (II) suivante : 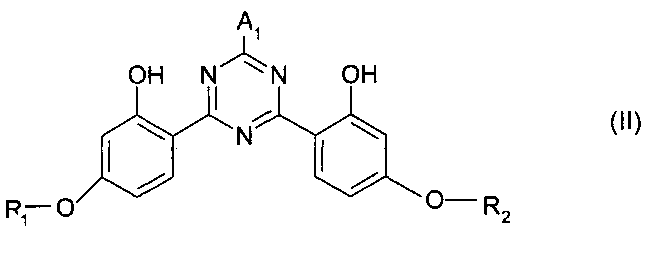 dans laquelle :
(i) les radicaux R₁ et R₂, identiques ou différents, désignent un radical alkyle en C₃-C₁₈ ; un radical alcényle en C₂-C₁₈ ou bien un reste de formule -CH₂-CH(OH)-CH₂-OT₁ où T₁ est un atome d'hydrogène ou un radical alkyle en C₁-C₈;
(ii) les radicaux R₁ et R₂, identiques ou différents, peuvent aussi désigner un reste de formule (1) suivante: 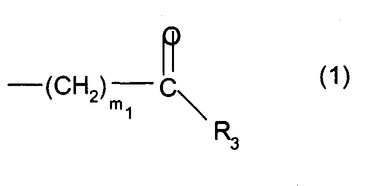 dans laquelle:
   - m₁ est un nombre de 1 à 3 ;
   - R₃ désigne un groupe hydroxy ; un radical alkyle en C₁-C₅ non substitué ou substitué par un ou plusieurs groupes hydroxy ; un radical alcoxy en C₁-C₅ ; un groupe amino; un radical mono- ou dialkyl(C₁-C₅)amino ; un cation métallique M ; un reste choisi parmi l'une des formules (2) à (7) suivantes : 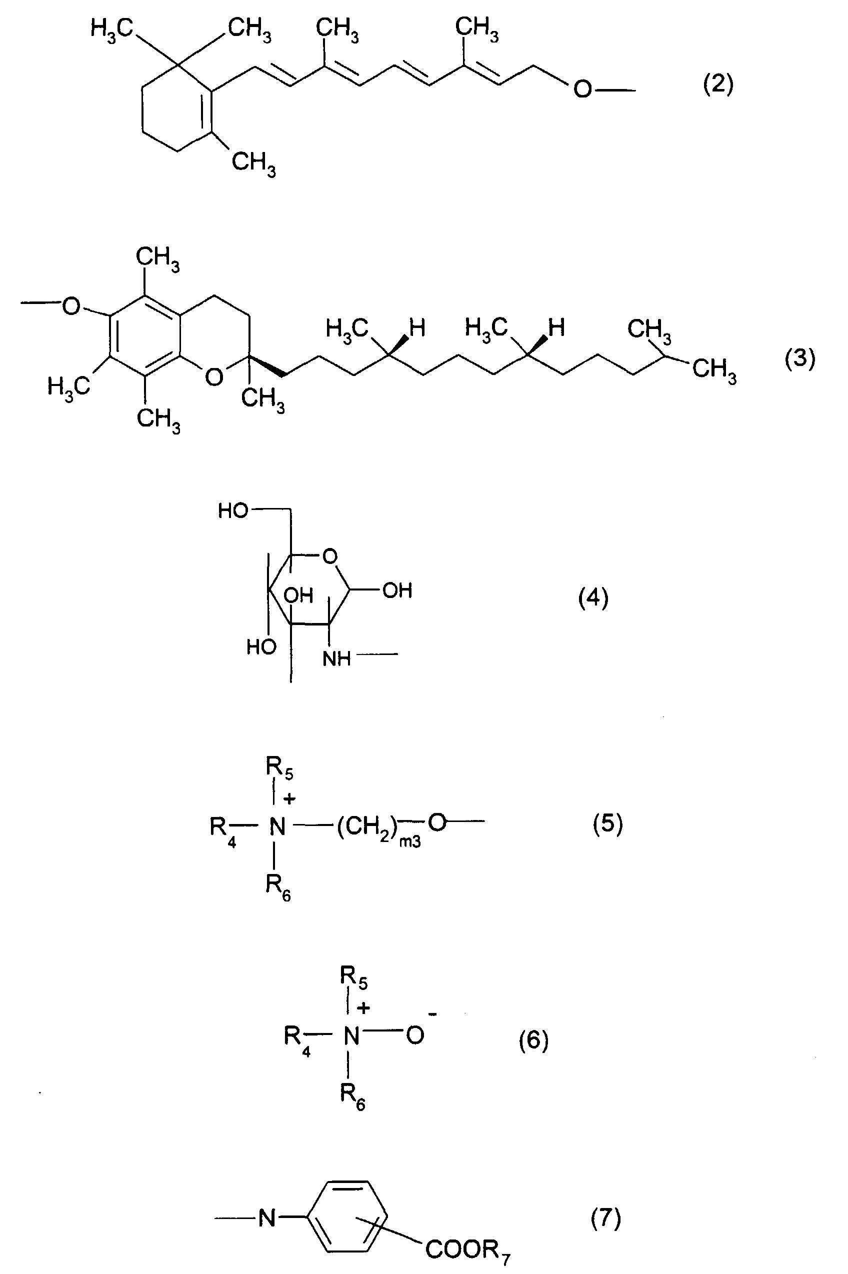 dans lesquelles
   - les radicaux R₄, R₅ et R₆, identiques ou différents, désignent un radical alkyle en C₁-C₁₄ non substitué ou substitué par un ou plusieurs groupes hydroxy ; m₃ est un nombre de 2 à 14 ;
   - R₇ représente un atome d'hydrogène, un cation métallique M, un radical alkyle en C₁-C₅ ou un reste de formule -(CH₂)ₘ₂-OT₁ où m₂ est un nombre de 1 à 4 et T₁ a la même signification indiquée ci-dessus ;
(iii) les radicaux R₁ et R₂, identiques ou différents, peuvent encore désigner un reste de formule (8) suivante : 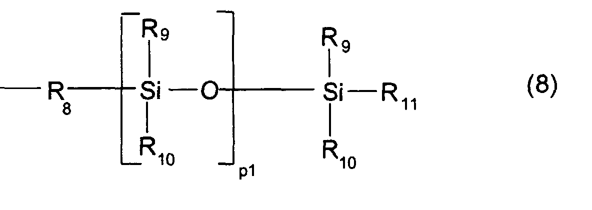 dans laquelle :
   - R₈ désigne une liaison covalente ; un radical alkyle linéaire ou ramifié en C₁-C₄ ou bien un reste de formule Cₘ₄H₂ₘ₄₋ ou -Cₘ₄H₂ₘ₄-O- où m₄ est un nombre de 1 à 4 ;
   - p₁ est un nombre de 0 à 5 ;
   - les radicaux R₉, R₁₀ et R₁₁, identiques ou différents, désignent un radical alkyle en C₁-C₁₈ ; un alcoxy en C₁-C₁₈ ou un reste de formule : 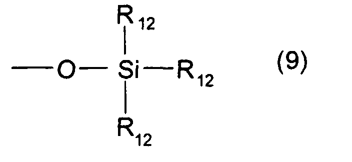 où R₁₂ est un radical alkyle en C₁-C₅ ;
   - A₁ désigne un reste répondant à l'une des formules suivantes : 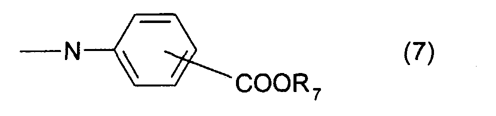 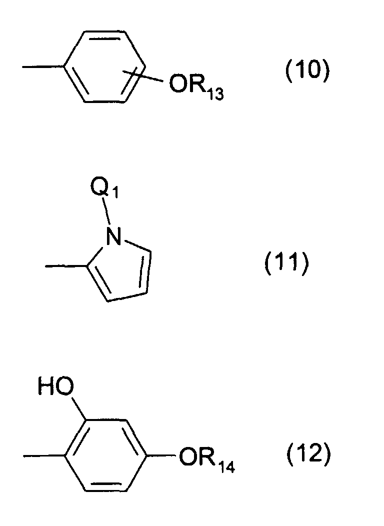 dans lesquelles :
   - R₇ a les mêmes significations indiquées ci-dessus ;
   - R₁₃ désigne un atome d'hydrogène, un radical alkyle en C₁-C₁₀, un radical de formule : -(CH₂CHR₁₆-O)ₙ₁R₇ où n₁ est un nombre de 1 à 16, R₁₆ est hydrogène ou méthyle ou bien un reste de structure -CH₂-CH-(OH)-CH₂OT₁ avec T₁ ayant la même signification indiquée ci-dessus.
   - Q₁ est un radical alkyle en C₁-C₁₈ ;
   - R₁₄ est un radical répondant à la formule (1) : 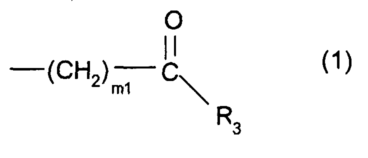 telle que définie ci-dessus.

Dans les formules (II) et (1) à (12) décrites ci-dessus :
- les radicaux alkyle sont linéaires ou ramifiés et peuvent être choisis par exemple parmi méthyle, éthyle, n-propyle, isopropyle, n-butyle, sec.butyle, tert.butyle, amyle, isoamyle, tert.amyle, heptyle, octyle, isooctyle, nonyle, décyle, undécyle, dodécyle, tétradécyle, pentadécyle, hexadécyle, heptadécyle ou octadécyle ;
- les radicaux alcényle peuvent être choisis par exemple parmi allyle, méthallyle, isopropènyle, 2-butényle, 3-butényle, isobutényle, n-penta-2,4-diènyle, 3-méthyl-but-2-enyle, n-oct-2-enyle, n-dodec-2-enyle, iso-dodecenyle, n-octadec-4-enyle ;
- les radicaux alcoxy sont linéaires ou ramifiés et peuvent être choisis par exemple parmi méthoxy, éthoxy, n-propoxy, isopropoxy, n-butoxy, sec.-butoxy, tert.-butoxy, amyloxy, isoamyloxy ou tert.amyloxy ;
- les radicaux mono ou dialkylamino en C₁-C₅ peuvent être choisis par exemple parmi méthylamino, éthylamino, propylamino, n-butylamino, sec.butylamino, tert.butylamino, pentylamino, diméthylamino, diéthylamino, dibutylamino ou méthyléthylamino.
- les cations métalliques sont des cations alcalins, alcalino-terreux ou métalliques choisis par exemple parmi lithium, potassium, sodium, calcium, magnésium, cuivre et zinc.

Les dérivés de bis-résorcinyl triazine de formule (II) de l'invention sont des filtres déjà connus en soi. Ils sont décrits et préparés selon les synthèses indiquées dans les demandes de brevet EP-A-0775 698 et EP-A-0878 469 (faisant partie intégrante du contenu de la description).

A titre d'exemples de composés bis-résorcinyl triazine de formule (II) utilisables, on peut citer ceux pour lesquels le radical A₁ désigne para-méthoxyphényle ou para-éthoxyphényle et les radicaux R₁ et R₂, identiques ou différents, désignent un radical de structure : 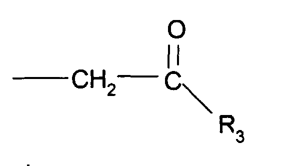 dans laquelle R₃ est choisi parmi :
- tert.butyloxy ;
- OH ;
- OM où M est un cation alcalin, alcalinoterreux ou choisi parmi le cuivre, le magnésium ou le zinc ;
- un groupe de structure 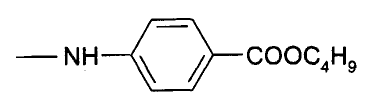 :
- un groupe de structure O⁻N⁺(CH₂CH₂OH)₃ ;
- un groupe de structure : 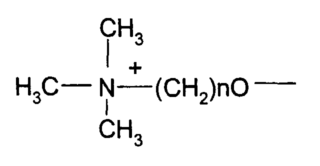 avec n variant de 2 à 16 ;
- un groupe de structure : 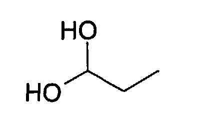
- un groupe de structure :

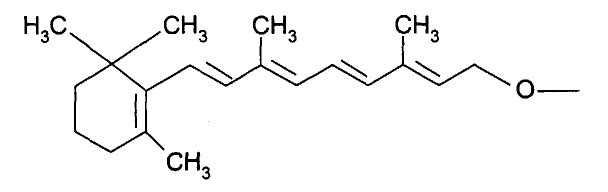

On peut également mentionner le composé bis-résorcinyl triazine répondant à la formule (II) dans laquelle le radical A₁ désigne para-hydroxyphényle et les radicaux R₁ et R₂ désignent simultanément un groupe de structure :

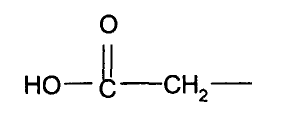

A titre d'exemples de composés de formule (II) utilisables, on peut citer :
- la 2,4-bis {[4-(2-éthyl-hexyloxy)-2-hydroxy]-phényl}-6-(4-méthoxy-phenyl)-1,3,5-triazine ;
- la 2,4-bis{[4-(3-(2-propyloxy)-2-hydroxy-propyloxy)-2-hydroxy]-phényl}-6-(4-méthoxyphényl)-1,3,5-triazine ;
- la 2,4-bis {[4-(2-éthyl-hexyloxy)-2-hydroxy]-phényl}-6-[4-(2-méthoxyéthyl-carboxyl)-phénylamino]-1,3,5-triazine ;
- la 2,4-bis {[4-tris(triméthylsiloxy-silylpropyloxy)-2-hydroxy]-phényl}-6-(4-méthoxyphényl)-1,3,5-triazine ;
- la 2,4-bis {[4-(2''-méthylpropenyloxy)-2-hydroxy]-phényl}-6-(4-méthoxyphényl)-1,3,5-triazine ;
- la 2,4-bis {[4-( 1',1',1', 3',5',5',5'-heptaméthyltrisiloxy-2''-méthylpropyloxy)-2-hydroxyl]-phényl}-6-(4-méthoxyphényl)-1,3,5-triazine.
- la 2,4-bis{[4-(3-(2-propyloxy)-2-hydroxy-propyloxy)-2-hydroxy]-phényl}-6-[4-(éthoxycarbonyl)-phénylamino]-1,3,5-triazine ;
- la 2,4-bis {[4-(2-éthylhexyloxy)-2-hydroxy]-phényl}-6-(1-méthylpyrrol-2-yl)-1,3,5-triazine.

Les composés dérivés de bis-résorcinyl triazine de formule (II) plus particulièrement préférés selon l'invention sont choisis dans le groupe constitué par :
- la 2,4-bis {[4-(2-éthylhexyloxy)-2-hydroxy]-phényl}-6-(4-méthoxy-phényl)-1,3,5-triazine ;
- la 2,4-bis {[4-tris(triméthylsiloxy)silylpropyloxy)-2-hydroxy]-phenyl}-6-(4-méthoxyphényl)-1,3,5-triazine ;
- la 2,4-bis {[4-( 1',1',1',3',5',5',5'-heptaméthyltrisiloxy-2''-méthylpropyloxy)-2-hydroxy]-phényl}-6-(4-méthoxyphényl)-1,3,5-triazine.

Le ou les filtres organiques du type dérivé de bis-résorcinyl triazine sont généralement présents dans les compositions filtrantes selon l'invention à une concentration totale comprise entre 0,1 et 15 % en poids environ, et de préférence entre 0,2 et 10 % en poids environ, par rapport au poids total de la composition.

Parmi les composés comportant au moins deux groupes benzoazolyle conformes à l'invention, on utilisera de préférence ceux répondant à la formule générale (III) suivante : 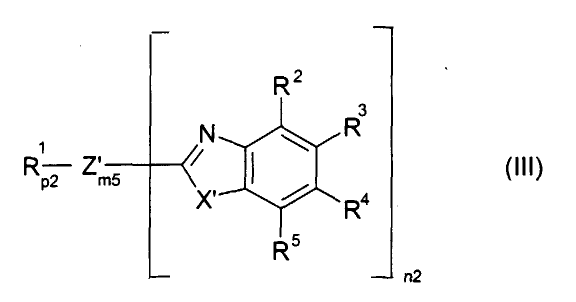 dans laquelle :
- Z' représente un reste organique de valence (p₂ + n₂) comportant une ou plusieurs doubles liaisons placées de telle sorte qu'elle complète le système de doubles liaisons d'au moins deux groupes benzoazolyle tels que définis à l'intérieur des crochets pour former un ensemble totalement conjugué ;
- X' désigne S, O ou NR⁶
- R¹ désigne hydrogène, alkyle en C₁-C₁₈, alcoxy en C₁-C₄, un aryle en C₅-C₁₅, un acyloxy en C₂-C₁₈, SO₃Y' ou COOY' ;
- les radicaux R², R³, R⁴ et R⁵, identiques ou différents, désignent un groupe nitro ou ont les mêmes significations que R¹ ;
- R⁶ désigne hydrogène, un alkyle en C₁-C₄ ou un hydroxyalkyle en C₁-C₄ ;
- Y' désigne hydrogène, Li, Na, K, NH₄, 1/2Ca, 1/2Mg, 1/3Al ou un cation résultant de la neutralisation d'un groupe acide libre par une base azotée organique;
- m₅ est 0 ou 1 ;
- n₂ est un nombre de 2 à 6 ;
- p₂ est un nombre de 1 à 4 ;
- sous réserve que P₂ + n₂ ne dépasse pas la valeur 6.

Les composés de formule (III) conformes à l'invention sont des filtres UV-A hydrosolubles déjà connus dans la demande de brevet EP-A-0669323. Ils sont décrits et préparés selon les synthèses indiquées dans le brevet US 2,463,264 ainsi que la demande de brevet EP-A-0669323 (ces deux documents faisant partie intégrante du contenu de la description).

Parmi les composés de formule (III) de l'invention, on préfère ceux pour lesquels le groupe Z' est choisi dans le groupe constitué par :
(a) un radical hydrocarboné en C₂-C₆ aliphatique linéaire insaturé pouvant être interrompu par un groupe aryle en C₅-C₁₂ ou un hétéroaryle en C₄-C₁₀ comme par exemple :
   -CH=CH-, -CH=CH-CH=CH- ou bien 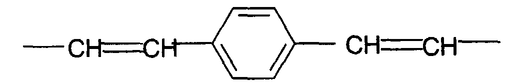
(b) un groupe aryle en C₅-C₁₅ pouvant être interrompu par un radical hydrocarboné en C₂-C₆ aliphatique linéaire insaturé comme par exemple les groupes suivants : 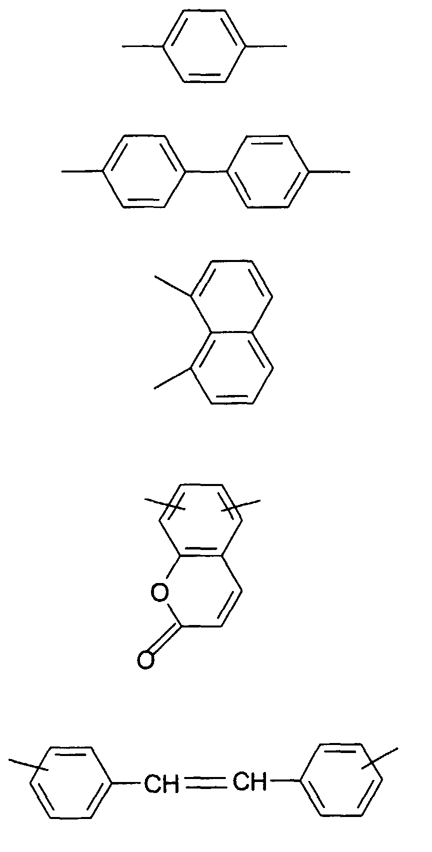
(c) un reste hétéroaryle en C₃-C₁₀ comme par exemple les groupes suivants : 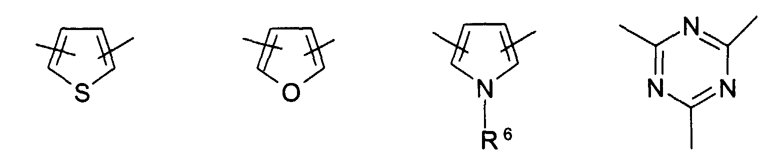 où R⁶ a la même signification indiquée ci-dessus; lesdits radicaux Z' tels que définis dans les paragraphes (a), (b) et (c) pouvant être substitués par des radicaux alkyle en C₁-C₆, alcoxy en C₁-C₆, phénoxy, hydroxy, méthylènedioxy ou amino éventuellement substitués par un ou 2 radicaux alkyle en C₁-C₅.

A titre d'exemples de composés de formule (III) utilisables, on peut citer ceux de structure suivante ainsi que leurs sels :

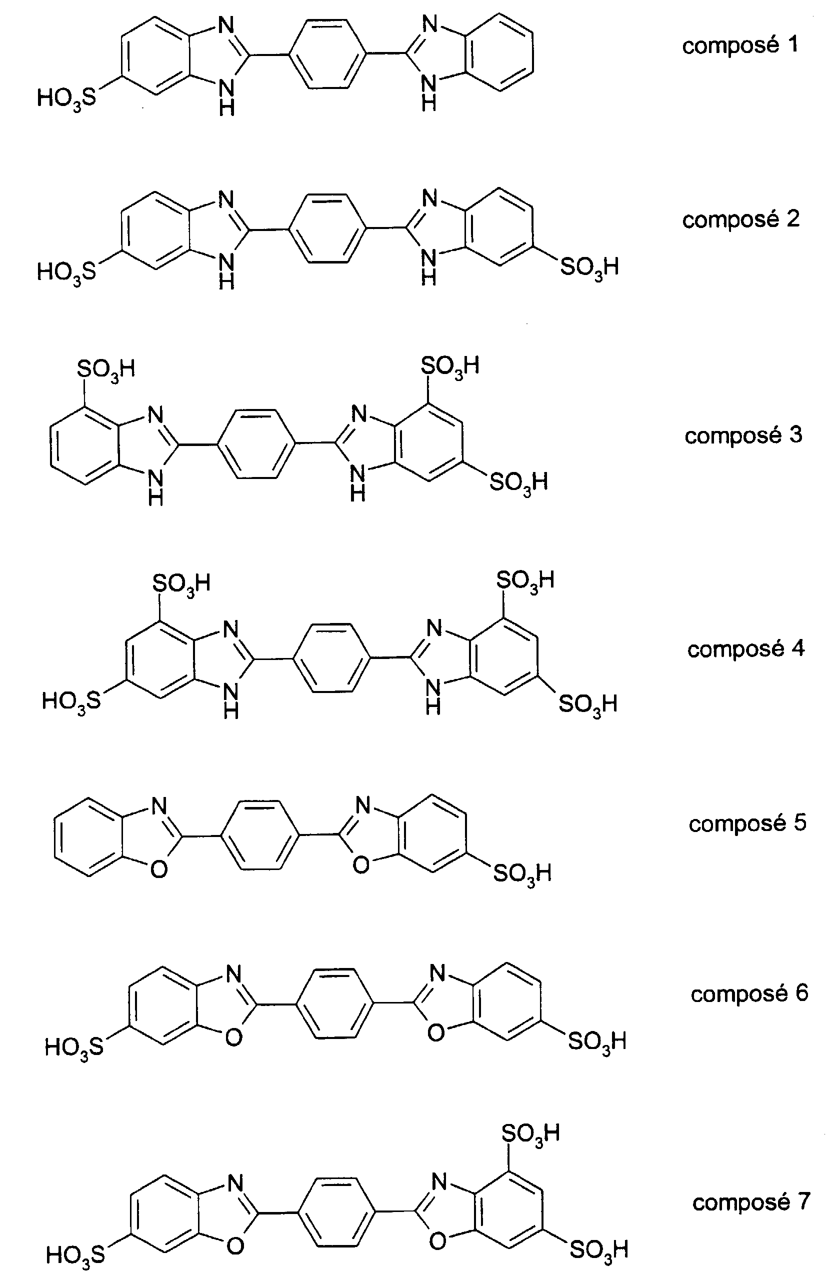

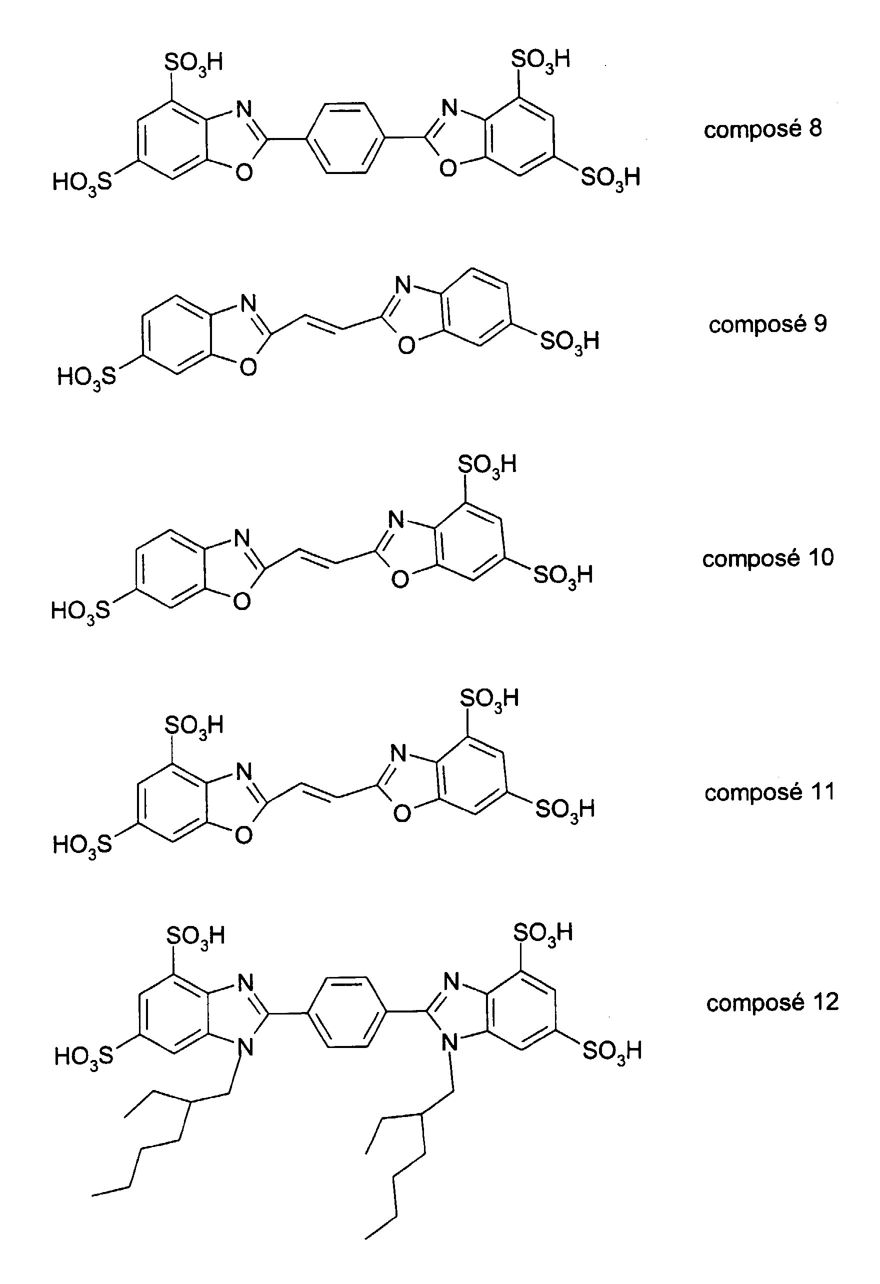

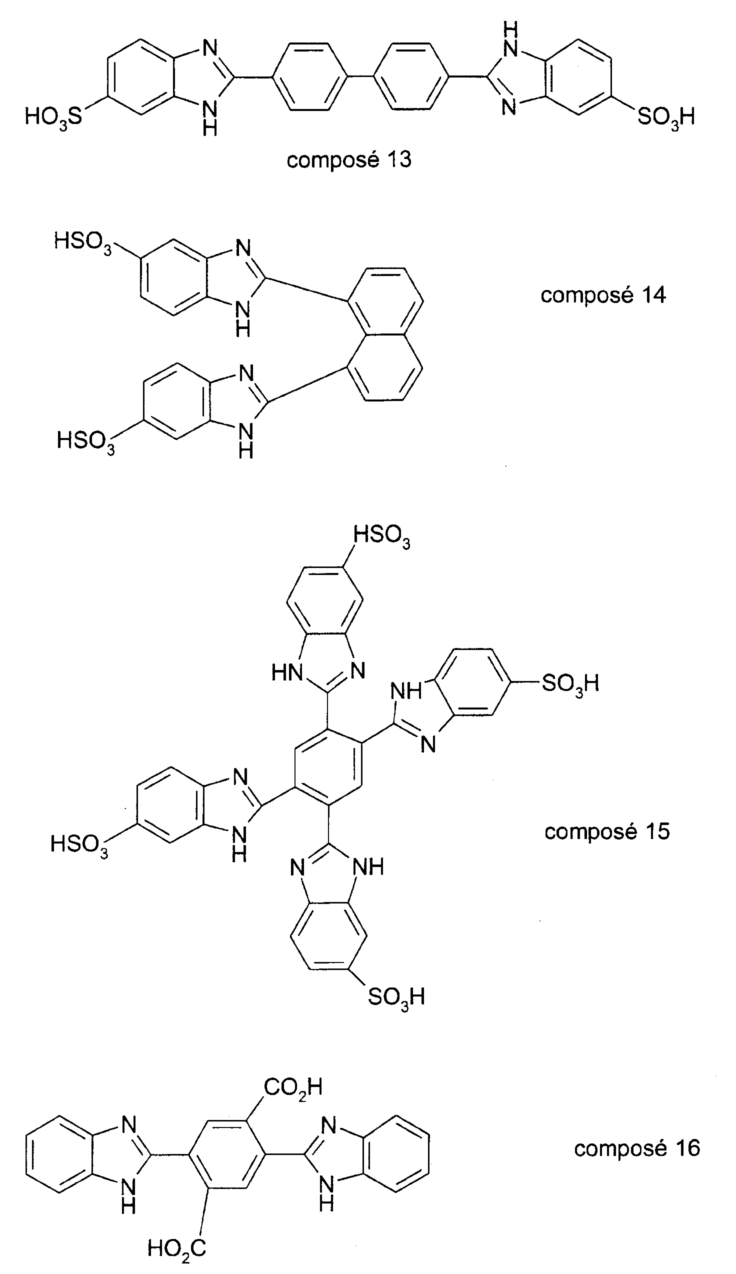

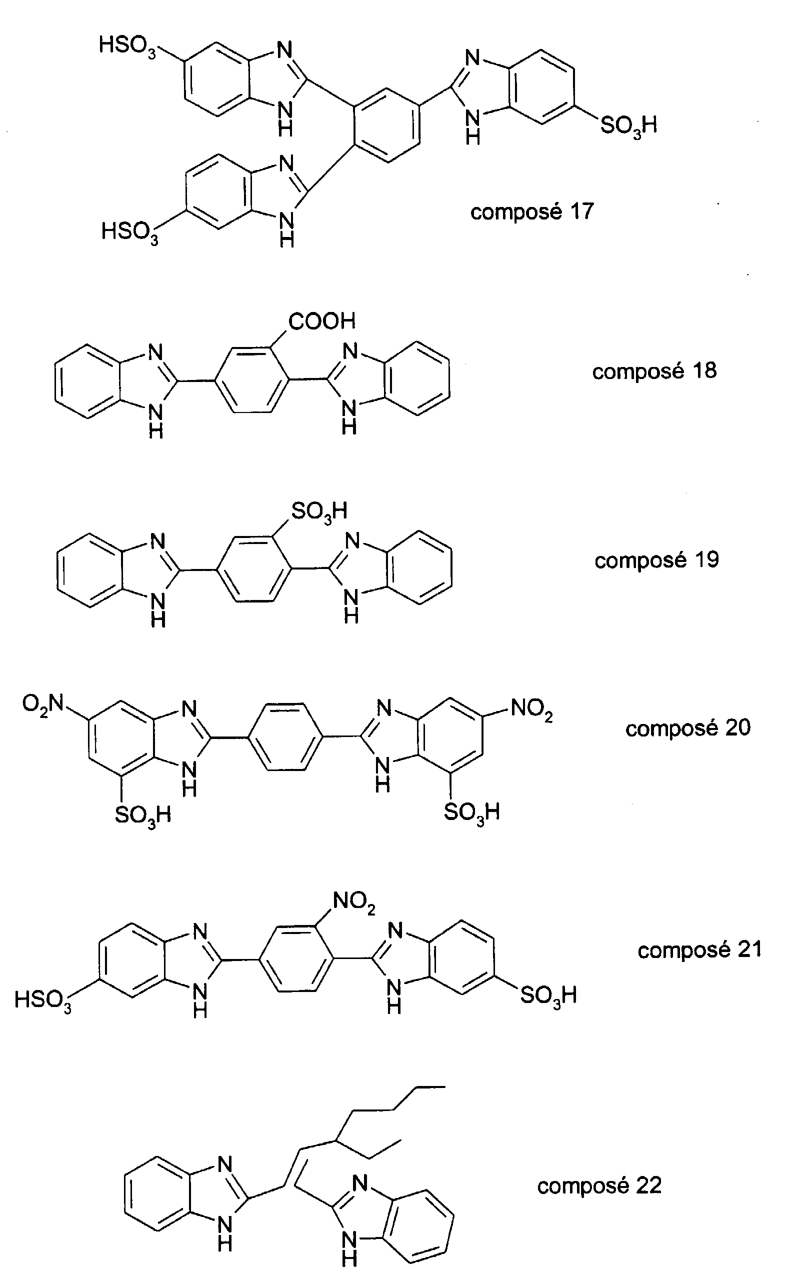

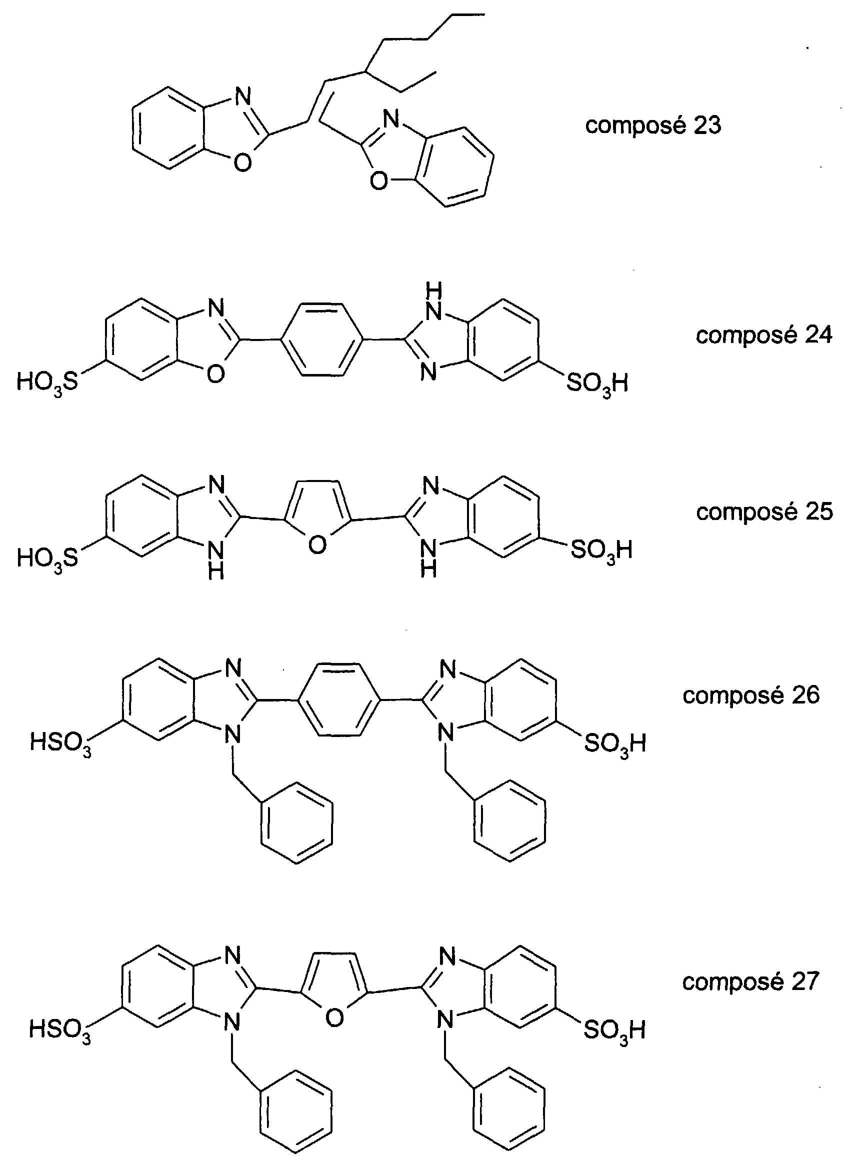

Parmi tous ces composés on préférera tout particulièrement l'acide 1,4-bis-benzimidazolyl-phènylèn-3,3' ,5,5'-tétrasulfonique (composé 4) ainsi que ses sels de stucture suivante:

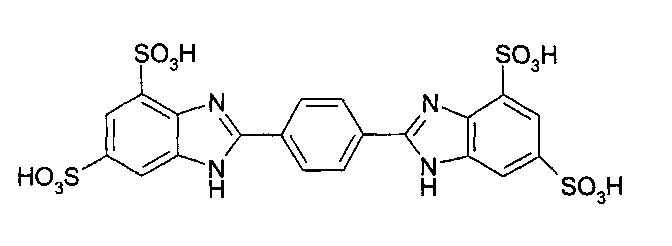

Comme autres exemples de composés comportant au moins deux groupes benzoazolyle, on peut citer également les composés suivants ainsi que leurs sels :

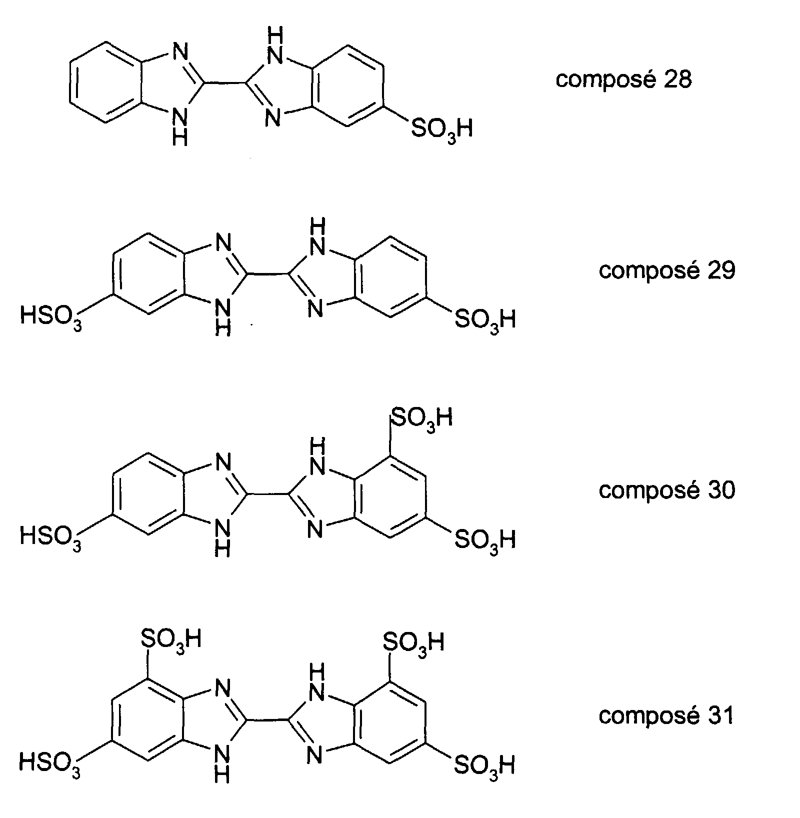

Comme exemples de composés comportant au moins un groupe benzodiazolyle utilisables selon l'invention, on peut mentionner en particulier les composés suivants ainsi que leurs sels :

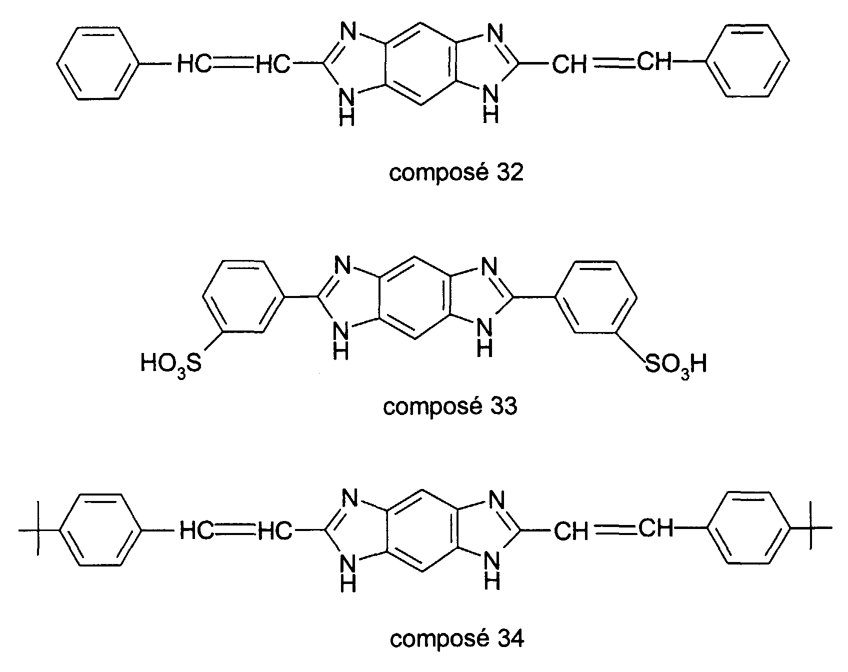

Le ou les composés à groupements benzoazolyle ou benzodiazolyle conformes à l'invention peuvent être présents dans les compositions selon l'invention à une concentration comprise entre 0,1 et 15%, de préférence entre 0,2 et 10%, en poids par rapport au poids total de la composition.

Comme indiqué précédemment, selon une caractéristique essentielle de la présente invention, il convient que les trois types de filtres solaires soient tous présents dans la composition finale dans une proportion respective telle qu'un effet de synergie au niveau de l'indice de protection conféré par l'association résultante, soit obtenu de manière notable, substantielle et significative.

En outre, et d'une manière générale, on notera que les concentrations et rapports en acide benzène 1,4-di(3-méthylidène-10-camphosulfonique), en dérivé de bis-résorcinyl triazine et en composé à groupements benzoazolyle ou benzodiazolyle tels que définis précédemment sont choisis de manière telle que l'indice de protection solaire de la composition finale soit de préférence d'au moins 2.

Selon un mode préféré de réalisation de la présente invention, le support cosmétiquement acceptable dans lequel se trouvent contenus les différents types de filtres est une émulsion de type huile-dans-eau.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles, autres bien sûr que les deux filtres mentionnés ci-avant. Ces filtres complémentaires peuvent être notamment choisis parmi les dérivés cinnamiques ; les dérivés salicyliques, les dérivés du camphre ; les dérivés de triazine autres que ceux définis précédemment tels que ceux décrits dans les demandes de brevet EP863145, EP517104, EP570838 et EP796851 ; les dérivés de la benzophénone; les dérivés du dibenzoylméthane ; les dérivés de β,β'-diphénylacrylate, les dérivés de benzimidazole autres que ceux décrits précédemment ; les dérivés de l'acide p-aminobenzoïque; les polymères filtres et silicones filtres tels que ceux décrits dans la demande WO-93/04665.

Comme exemples de filtres solaires complémentaires actifs dans l'UV-A et/ou l'UV-B, on peut citer :
l'acide p-aminobenzoïque,
le p-aminobenzoate oxyéthyléné (25mol),
le p-diméthylaminobenzoate de 2-éthylhexyle,
le p-aminobenzoate d'éthyle N-oxypropyléné
le p-aminobenzoate de glycérol,
le salicylate d'homomenthyle,
le salicylate de 2-éthylhexyle,
le salicylate de triéthanolamine,
le salicylate de 4-isopropylbenzyle,
le 4-ter-butyl-4'-méthoxy-dibenzoylméthane,
le 4-isopropyl-dibenzoylméthane,
le 4-méthoxy cinnamate de 2-éthylhexyle,
le diisopropyl cinnamate de méthyle,
le 4-méthoxy cinnamate d'isoamyle,
le 4-méthoxy cinnamate de diéthanolamine,
l'anthranilate de menthyle,
le 2-éthyl hexyl-2-cyano-3,3'-diphénylacrylate,
l'éthyl-2-cyano-3,3'-diphénylacrylate,
l'acide 2-phényl benzimidazole 5-sulfonique et ses sels,
le 3-(4'-triméthylammonium)-benzylidèn-bornan-2-on-méthylsulfate,
le 2-hydroxy-4-méthoxybenzophénone,
le 2-hydroxy-4-méthoxybenzophénone-5-sulfonate,
le 2,4-dihydroxybenzophénone,
le 2,2',4,4'-tétrahydroxybenzophénone,
le 2,2'-dihydroxy-4,4'diméthoxybenzophénone,
le 2-hydroxy-4-n-octoxybenzophénone,
le 2-hydroxy-4-méthoxy-4'-méthylbenzophénone,
l'acide urocanique,
l'acide α-(2-oxoborn-3-ylidène )-tolyl-4-sulfonique et ses sels
le 3-(4'-sulfo)benzylidèn-bornan-2-one et ses sels,
le 3-(4'méthylbenzylidène)-d,l-camphre,
le 3-benzylidéne-d,l-camphre,
la 2,4,6-tris-[ p-(2'-éthylhexyl-1'-oxycarbonyl)anilino]-1,3,5-triazine,
2-[p-(tertiobutylamido)anilino]-4, 6-bis-[(p-(2'-éthylhexyl-1'-oxycarbonyl)anilino]-1,3,5-triazine,
le polymère de N-[(2 et 4)-[(2-oxoborn-3-ylidèn)méthyl] benzyl]-acrylamide,
Le drométrizole trisiloxane (nom INCI),
les polyorganosiloxanes à fonction malonate.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions de l'invention peuvent comprendre en outre des adjuvants cosmétiques classiques notamment choisis parmi les corps gras, les solvants organiques, les épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les homopolymères d'acide acrylique réticulés, les gommes de guar et celluloses modifiées ou non telles que la gomme de guar hydroxypropylée, la méthylhydroxyéthylcellulose, l'hydroxypropylméthyl cellulose ou encore l'hydroxyéthylcellulose.

Bien entendu, l'homme de l'art veillera à choisir ce ou ces éventuels composés complémentaires et/ou leurs quantités de manière telle que les propriétés avantageuses, en particulier les indices de protection solaire conférés, attachées intrinsèquement à l'association ternaire conforme à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie cidessus.

Des exemples concrets, mais nullement limitatifs, illustrant l'invention, vont maintenant être donnés.

### EXEMPLES

| **COMPOSITION** | **EX 1** |
|---|---|
| Mélange d'alcool cétylstéarylique et d'alcool cétylstéarylique oxyéthyléné (33 OE) 80/20 (SINNOWAX AO -HENKEL) | 7g |
| Mélange de mono et distéarate de glycérol (CERASYNT SD-V ISP) | 2g |
| Alcool cétylique | 1.5g |
| Polydiméthylsiloxane (DOW CORNING 200 FLUID -DOW CORNING) | 1g |
| Benzoate d'alcools en C₁₂/C₁₅ (WITCONOL TN -WITCO) | 15g |
| 2,4-bis-{[4-(2-ethyl-hexyloxy)-2-hydroxy]-phenyl}-6-(4-methoxyphenyl)-1,3,5-triazine | 2g |
| Glycérine | 15g |
| Acide 1,4-bis-benzimidazolyl-phènylène-3,3',5,5'-tétrasulfonique | 2g |
| Acide benzène 1,4-di(3-méthylidène-10-camphosulfonique), (MEXORYL SX-CHIMEX) | 2g |
| Triéthanolamine | qs pH 7 |
| Conservateurs | qs |
| Eau déminéralisée qsp | 100 g |

| **COMPOSITION** | **EX 2** |
|---|---|
| Mélange mono /distéarate de glycerol / stéarate de polyéthyléne glycol (100 OE) (ARLACEL 165 FL - ICI) | 2g |
| Alcool stéarylique (LANETTE 18- HENKEL) | 1g |
| Acide stéarique d'huile de palme (STEARINE TP - STEARINERIE DUBOIS) | 2.5g |
| Poly diméthylsiloxane (DOW CORNING 200 FLUID - DOW CORNING) | 0.5g |
| Benzoate d'alcools en C₁₂/C₁₅ (WITCONOL TN -WITCO) | 20g |
| Triéthanolamine | 0.5g |
| 2,4-Bis-{[4-tris(trimethylsiloxy-silylpropyloxy)-2-hydroxy]-phenyl}-6-(4-methoxyphenyl)-1,3,5-triazine | 2.5g |
| Glycérine | 5g |
| Phosphate d'alcool hexadécylique,sel de potassium (AMPHISOL K - HOFFMAN LAROCHE) | 1g |
| Acide polyacrylique (SYNTHALEN K - 3V) | 0.3g |
| Hydroxypropyl méthyl cellulose (METHOCEL F4M -DOW CHEMICAL) | 0.1g |
| Acide 1,4-bis-benzimidazolyl-phènylène-3,3',5,5'-tétrasulfonique | 1.5g |
| Acide benzène 1,4-di(3-méthylidène-10-camphosulfonique), (MEXORYL SX-CHIMEX) | 1.5g |
| Triéthanolamine | qs pH 7 |
| Conservateurs | qs |
| Eau démineralisée qsp | 100 g |

| **COMPOSITION** | **EX 3** |
|---|---|
| Mélange d'alcool cétylstéarylique et d'alcool cétylstéarylique oxyéthyléné (33 OE) 80/20 (SINNOWAX AO -HENKEL) | 7g |
| Mélange de mono et distéarate de glycérol (CERASYNT SD-V ISP) | 2g |
| Alcool cétylique | 1.5g |
| Polydiméthyl siloxane (DOW CORNING 200 FLUID -DOW CORNING) | 1g |
| Benzoate d'alcools en C₁₂/C₁₅ (WITCONOL TN -WITCO) | 15g |
| 2,4-Bis-{[4-(1',1',1',3',5',5',5'-Heptamethyltrisiloxy-2''-methyl-propyloxy)-2-hydroxy]-phenyl}-6-(4-methoxyphenyl)- 1,3,5-triazine | 2g |
| Glycérine | 15g |
| Acide 1,4-bis-benzimidazolyl-phènylène-3,3',5,5'-tétrasulfonique | 1.5g |
| Acide benzène 1,4-di(3-méthylidène-10-camphosulfonique), (MEXORYL SX-CHIMEX) | 2g |
| Triéthanolamine | qs pH 7 |
| Conservateurs | qs |
| Eau déminéralisée qsp | 100 g |

## Revendications

1. Composition cosmétique à usage topique, en particulier pour la photoprotection de la peau et/ou des cheveux, caractérisée par le fait qu'elle comprend, dans un support cosmétiquement acceptable:
(a) de l'acide benzène 1 ,4-di(3-méthylidène-10-camphosulfonique), éventuellement sous forme partiellement ou totalement neutralisée, à titre de premier filtre,
(b) à titre de second filtre, au moins un dérivé de bis-résorcinyl triazine;
(c) à titre de troisième filtre, au moins un composé comportant par molécule au moins deux groupes benzoazolyle ou un composé comportant par molécule au moins un groupe benzodiazolyle;
lesdits premier, second et troisième filtres étant présents dans une proportion produisant une activité synergique au niveau des indices de protection solaire conférés.

2. Composition selon la revendication 1, où le dérivé de bis-résorcinyl triazine répond à la formule (II) suivante: 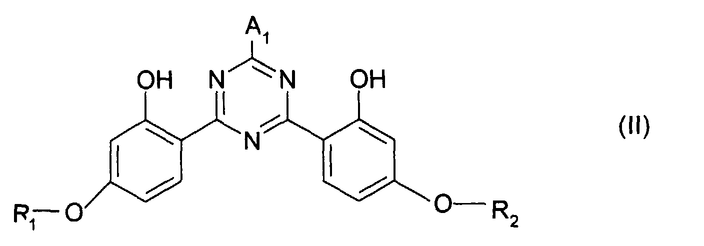 dans laquelle:
(i) les radicaux R₁ et R₂, identiques ou différents, désignent un radical alkyle en C₃-C₁₈ ; un radical alcényle en C₂-C₁₈ ou bien un reste de formule -CH₂-CH(OH)-CH₂-OT₁ où T₁ est un atome d'hydrogène ou un radical alkyle en C₁-C₈;
(ii) les radicaux R₁ et R₂, identiques ou différents, peuvent aussi désigner un reste de formule (1) suivante: 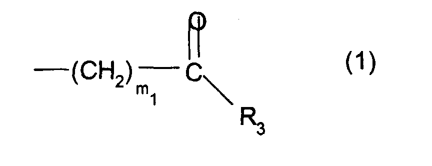 dans laquelle:
- m₁ est un nombre de 1 à 3;
- R₃ désigne un groupe hydroxy; un radical alkyle en C₁-C₅ non substitué ou substitué par un ou plusieurs groupes hydroxy; un radical alcoxy en C₁-C₅; un groupe amino; un radical mono- ou dialkyl(C₁-C₅)amino; un cation métallique; un reste choisi parmi l'une des formules (2) à (7) suivantes: 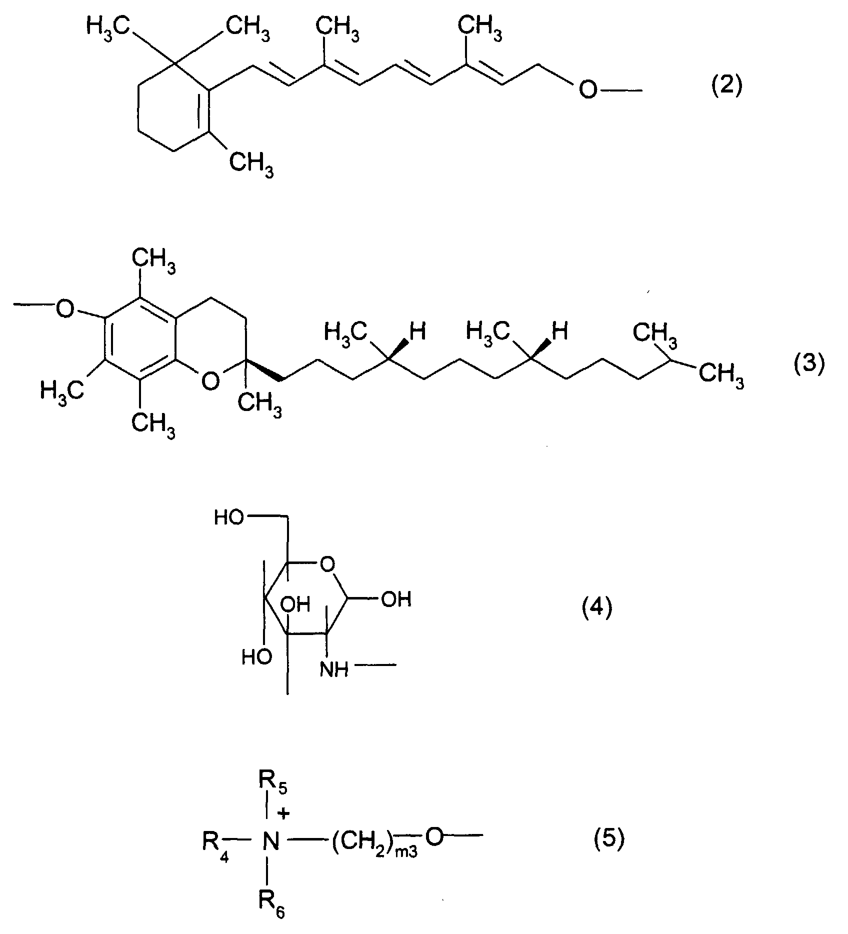 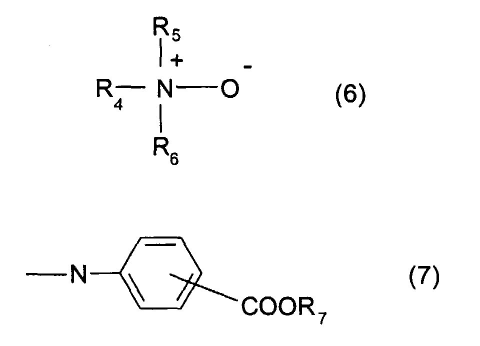 dans lesquelles
- les radicaux R₄, R₅ et R₆, identiques ou différents, désignent un radical alkyle en C₁-C₁₄ non substitué ou substitué par un ou plusieurs groupes hydroxy; m₃ est un nombre de 2 à 14;
- R₇ représente un atome d'hydrogène, un cation métallique, un radical alkyle en C₁-C₅ ou un reste de formule -(CH2)ₘ₂-OT₁ où m₂ est un nombre de 1 à 4 et T₁ a la même signification indiquée ci-dessus;
(iii) les radicaux R₁ et R₂, identiques ou différents, peuvent encore désigner un reste de formule (8) suivante: 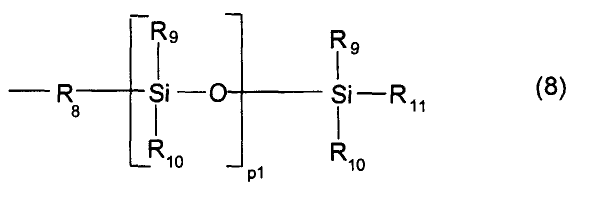 dans laquelle:
- R₈ désigne une liaison covalente; un radical alkyle linéaire ou ramifié en C₁-C₄ ou bien un reste de formule Cₘ₄H₂ₘ₄₋ou -Cₘ₄H₂ₘ₄-O- où m₄ est un nombre de 1 à 4;
- P₁ est un nombre de 0 à 5;
- les radicaux R₉, R₁₀ et R₁₁, identiques ou différents, désignent un radical alkyle en C₁-C₁₈; un alcoxy en C₁-C₁₈ ou un reste de formule: 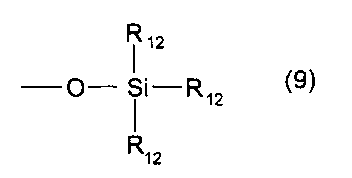 où R₁₂ est un radical alkyle en C₁-C₅;
- A₁ désigne un reste répondant à l'une des formules suivantes: 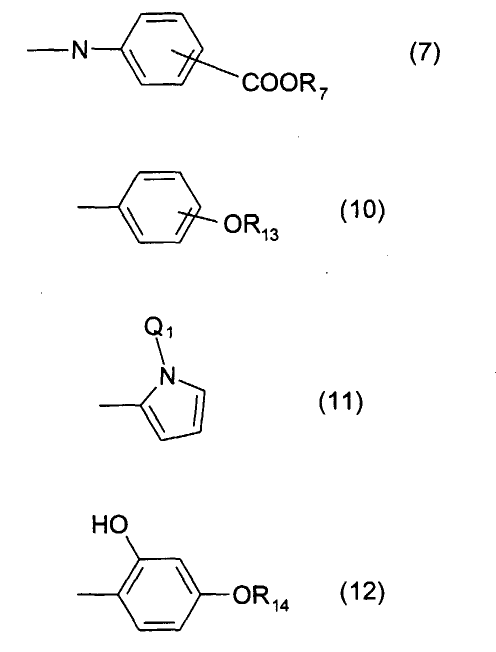 dans lesquelles:
- R₇ a les mêmes significations indiquées ci-dessus;
- R₁₃ désigne un atome d'hydrogène, un radical alkyle en C₁-C₁₀, un radical de formule: -(CH₂CHR₁₆-O)ₙ₁R₇ où n₁ est un nombre de 1 à 16, R₁₆ est hydrogène ou méthyle ou bien un reste de structure -CH₂-CH-(OH)-CH₂OT₁ avec T₁ ayant la même signification indiquée ci-dessus.
- Q₁ est un radical alkyle en C₁-C₁₈;
- R₁₄ est un radical répondant à la formule (1): 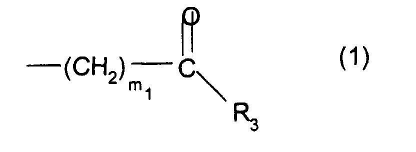 telle que définie ci-dessus.

3. Composition selon la revendication 2, caractérisée par le fait que le composé de formule (II) est choisi parmi:
(a) ceux pour lesquels le radical A₁ désigne para-méthoxyphényle ou paraéthoxyphényle et les radicaux R₁ et R₂, identiques ou différents, désignent un radical de structure: 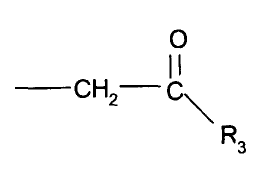 dans laquelle R₃ est choisi parmi:
- tert.butyloxy;
- OH;
- OM où M est un cation alcalin, alcalino-terreux ou choisi parmi le cuivre, le magnésium ou le zinc;
- un groupe de structure: 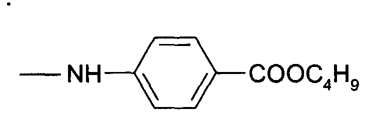
- un groupe de structure O⁻N⁺(CH₂CH₂OH)₃;
- un groupe de structure: 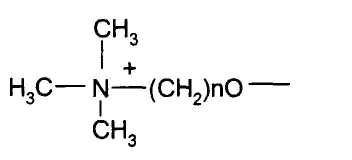 avec n variant de 2 à 16;
- un groupe de structure: 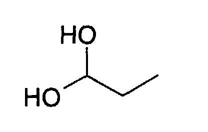
- un groupe de structure: 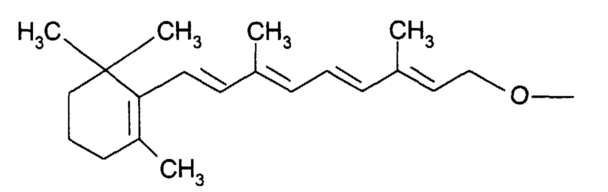
(b) le composé bis-résorcinyl triazine répondant à la formule (II) dans laquelle le radical A₁ désigne para-hydroxyphényle et les radicaux R₁ et R₂ désignent simultanément un groupe de structure :

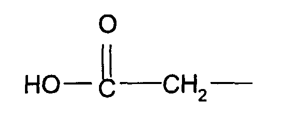

4. Composition selon la revendication 2, caractérisée par le fait que le composé de formule (II) est choisi dans le groupe constitué par:
- la 2,4-bis {[4-(2-éthyl-hexyloxy)-2-hydroxy]-phényl}-6-(4-méthoxy-phenyl)- 1,3,5-triazine;
- la 2 ,4-bis{[4-(3-(2-propyloxy)-2-hydroxy-propyloxy)-2-hydroxy]-phényl}-6-(4-méthoxyphényl)-1 ,3,5-triazine;
- la 2,4-bis {[4-(2-éthyl-hexyloxy)-2-hydroxy]-phényl}-6-[4-(2-méthoxyéthyl-carboxyl)-phénylamino]-1 ,3,5-triazine;
- la 2,4-bis {[4-(tris(triméthylsiloxy)silylpropyloxy)-2-hydroxy]-phényl}-6-(4-méthoxyphényl)-1 ,3,5-triazine;
- la 2,4-bis {[4-(2''-méthylpropenyloxy)-2-hydroxy]-phényl}-6-(4-méthoxyphényl)-1,3,5-triazine;
- la 2,4-bis {[4-( 1', 1', 1 ',3',5',5',5'-heptaméthyltrisiloxy-2''-méthylpropyloxy)-2-hydroxy]-phényl}-6-(4-méthoxyphényl)- 1,3 ,5-triazine.
- la 2 ,4-bis{[4-(3-(2-propyloxy)-2-hydroxy-propyloxy]-2-hydroxy]-phényl}-6-[4-(éthoxycarbonyl)-phénylamino]-1,3,5-triazine;
- la 2,4-bis {[4-(2-éthylhexyloxy)-2-hydroxy]-phényl}-6-( 1-méthylpyrrol-2-yl)- 1,3,5-triazine.

5. Composition selon la revendication 4, caractérisée par le fait que le composé de formule (II) est choisi dans le groupe constitué par:
- la 2,4-bis {[4-(2-éthylhexyloxy)-2-hydroxy]-phényl}-6-(4-méthoxy-phényl )- 1,3,5-triazine;
- la 2,4-bis {[4-(tris(triméthylsiloxy)silylpropyloxy)-2-hydroxy]-phenyl}-6-(4-méthoxyphényl)-1 ,3,5-triazine;
- la 2,4-bis {[4-( 1', 1', 1' ,3' ,5' ,5' ,5'-heptaméthyltrisiloxy-2''-méthylpropyloxy)-2-hydroxy]-phényl}-6-(4-méthoxyphényl )- 1,3, 5-triazine.

6. Composition selon l'une quelconque des revendications 1 à 5, caractérisée par le fait que la concentration en dérivé de bis-résorcinyl triazine est comprise entre 0,1 et 15% en poids par rapport au poids total de la composition.

7. Composition selon la revendication 6, caractérisée par le fait que ladite concentration est comprise entre 0,2 et 10% en poids par rapport au poids total de la composition.

8. Composition selon l'une quelconque des revendications 1 à 7, caractérisée par le fait que l'acide benzène 1,4-di(3-méthylidène-10-camphosulfonique), et ses sels répond à la formule (I) suivante: 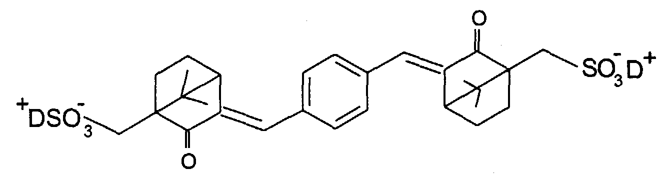 dans laquelle D désigne un atome d'hydrogène, un métal alcalin ou encore un radical NH(R₂₅)₃⁺ dans lequel les radicaux R₂₅, qui peuvent être identiques ou différents, désignent un atome d'hydrogène, un radical alkyle ou hydroxyalkyle en C₁-C₄ ou encore un groupement Mn⁺/n, Mⁿ⁺ désignant un cation métallique polyvalent dans lequel n est est égal à 2 ou 3 ou 4, Mⁿ⁺ désignant de préférence un cation métallique choisi parmi Ca²⁺, Zn²⁺, Mg²⁺, Ba²⁺, Al³⁺ et Zr⁴⁺ .

9. Composition selon l'une quelconque des revendications 1 à 8, caractérisée par le fait que l'acide benzène 1 ,4-di(3-méthylidène-10-camphosulfonique), ou l'un de ses sels est généralement présent à une concentration totale comprise entre 0,1 et 15% en poids environ par rapport au poids total de la composition.

10. Composition selon la revendication 9, où la concentration est comprise entre 0,2 et 10% en poids environ, par rapport au poids total de la composition

11. Composition selon l'une quelconque des revendications 1 à 10, caractérisée par le fait que le composé comportant au moins deux groupes benzoazolyle répond à la formule générale (III) suivante: 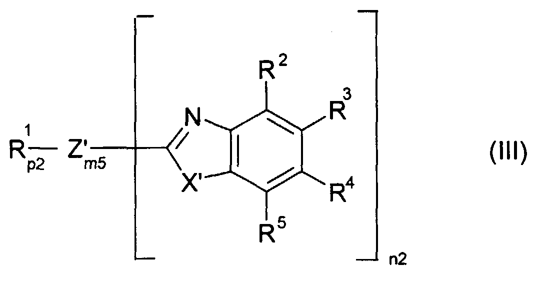 dans laquelle:
- Z' représente un reste organique de valence (p₂ + n₂) comportant une ou plusieurs doubles liaisons placées de telle sorte qu'elle complète le système de doubles liaisons d'au moins deux groupes benzoazolyle tels que définis à l'intérieur des crochets pour former un ensemble totalement conjugué;
- X' désigne S, O ou NR⁶
- R¹ désigne hydrogène, alkyle en C₁-C₁₈, alcoxy en C₁-C₄, un aryle en C₅-C₁₅, un acyloxy en C₂-C₁₈, SO₃Y' ou COOY';
- les radicaux R², R³, R⁴ et R⁵, identiques ou différents, désignent un groupe nitro ou ont les mêmes significations que R¹;
- R⁶ désigne hydrogène, un alkyle en C₁-C₄ ou un hydroxyalkyle en C₁-C₄;
- Y' désigne hydrogène, Li, Na, K, NH₄, 1/2Ca, 1/2Mg, 1/3Al ou un cation résultant de la neutralisation d'un groupe acide libre par une base azotée organique;
- m₅ est 0 ou 1 ;
- n₂ est un nombre de 2 à 6;
- p₂ est un nombre de 1 à 4;
- sous réserve que p₂ + n₂ ne dépasse pas la valeur 6.

12. Composition selon la revendication 11, où le radical Z' est choisi dans le groupe constitué par:
(a) un radical hydrocarboné en C₂-C₆ aliphatique linéaire insaturé pouvant être interrompu par un groupe aryle en C₅-C₁₂ ou un hétéroaryle en C₄-C₁₀
(b) un groupe aryle en C₅-C₁₅ pouvant être interrompu par un radical hydrocarboné en C₂-C₆ aliphatique linéaire insaturé ;
(c ) un groupe hétéroaryle en C₃-C₁₀; ledit radical Z' pouvant être substitué par des radicaux alkyle en C₁-C₆, alcoxy en C₁-C₆, phénoxy, hydroxy, méthylènedioxy ou amino, éventuellement substitués par un ou 2 radicaux alkyle en C₁-C₅.

13. Composition selon la revendication 11 ou 12, où le radical Z' est choisi dans le groupe constitué par les groupes suivants:
-CH=CH-
-CH=CH-CH=CH- 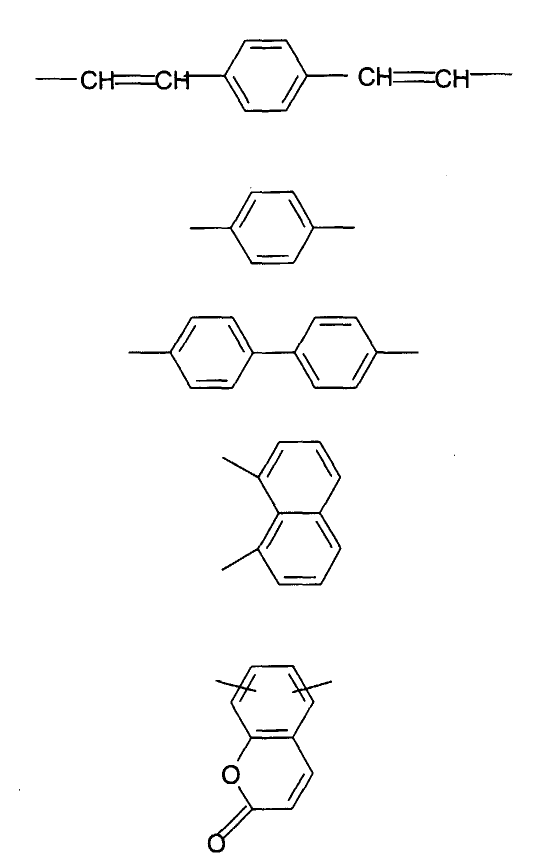 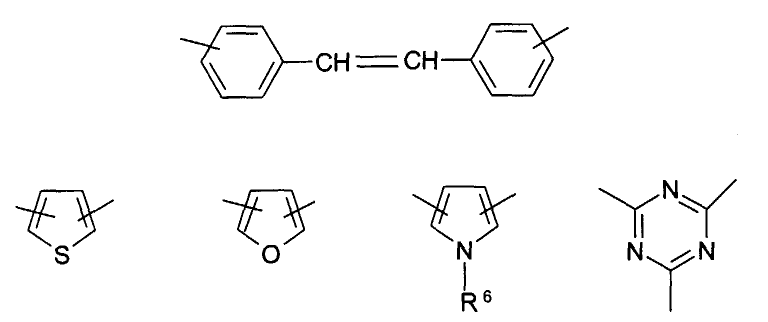
avec R⁶ ayant la même signification indiquée dans la revendication 10.

14. Composition selon l'une quelconque des revendications 11 à 13, où le composé de formule (III) est choisi parmi les composés suivants ou l'un de leurs sels:

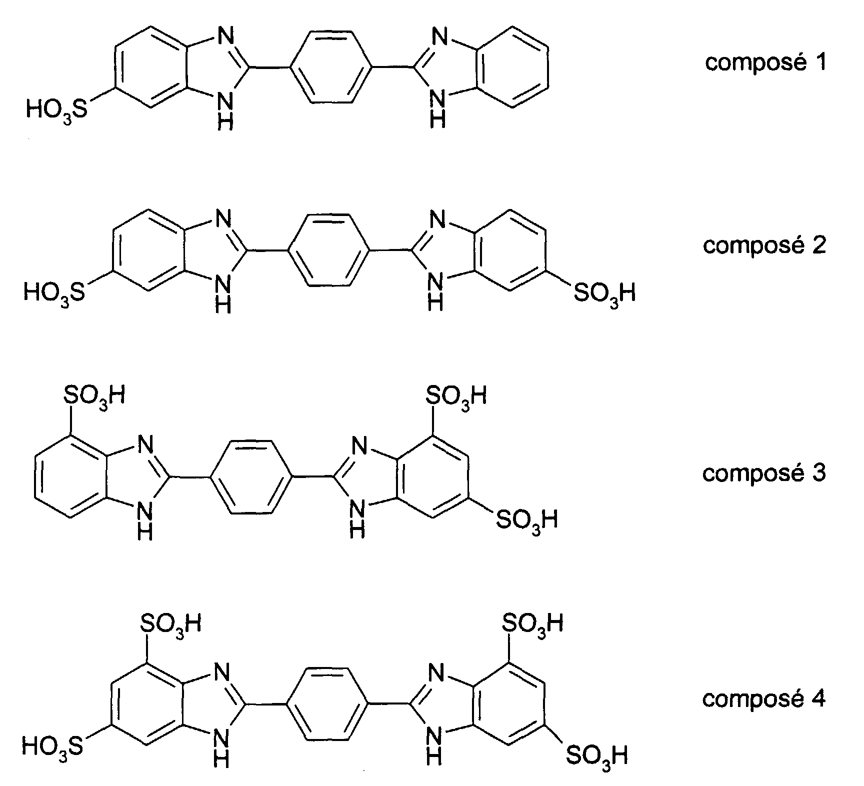

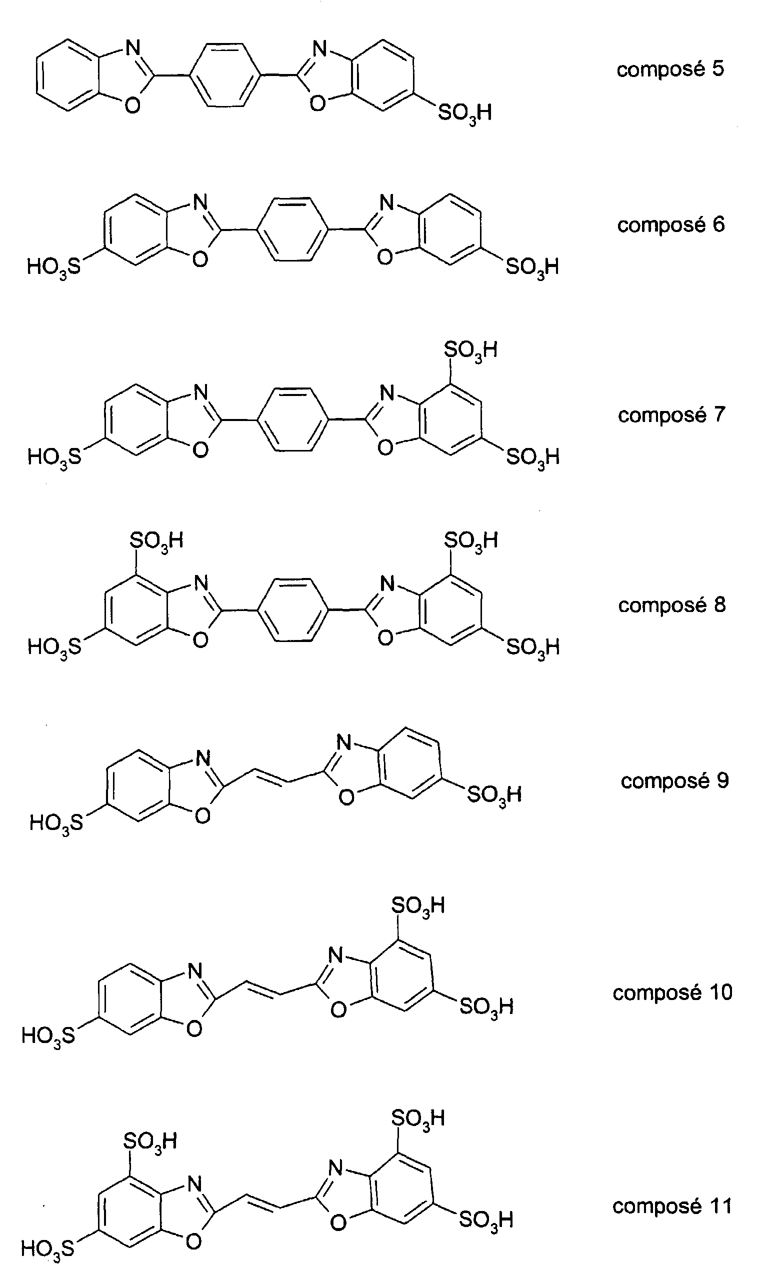

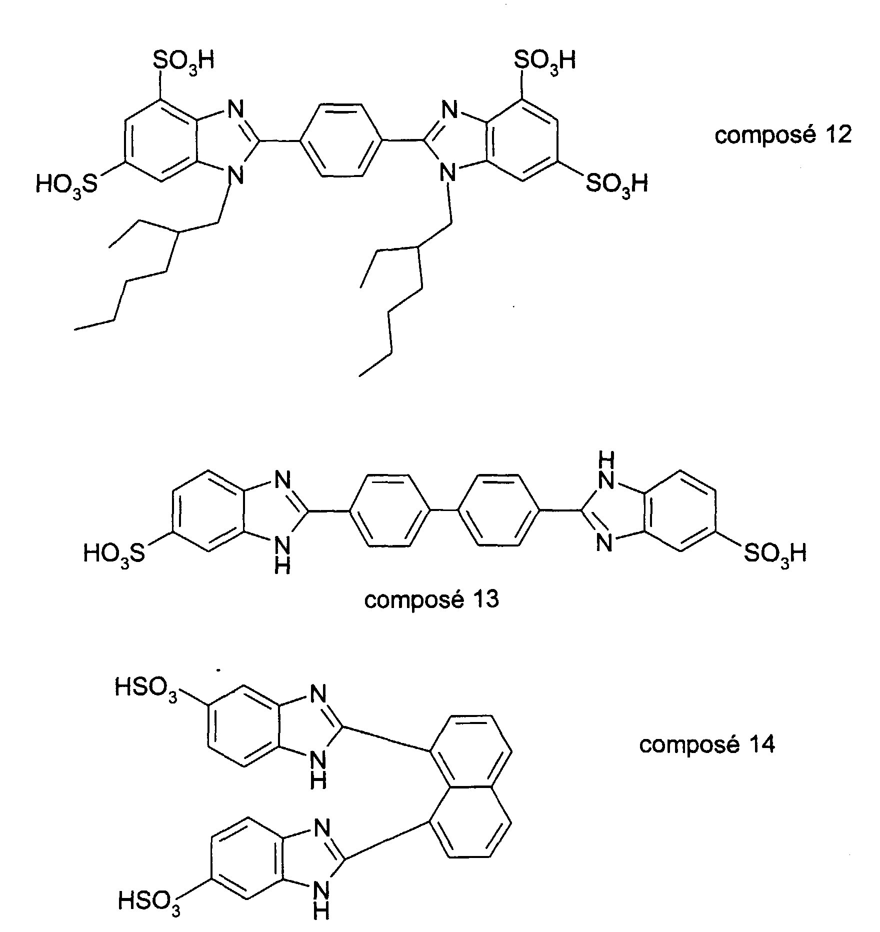

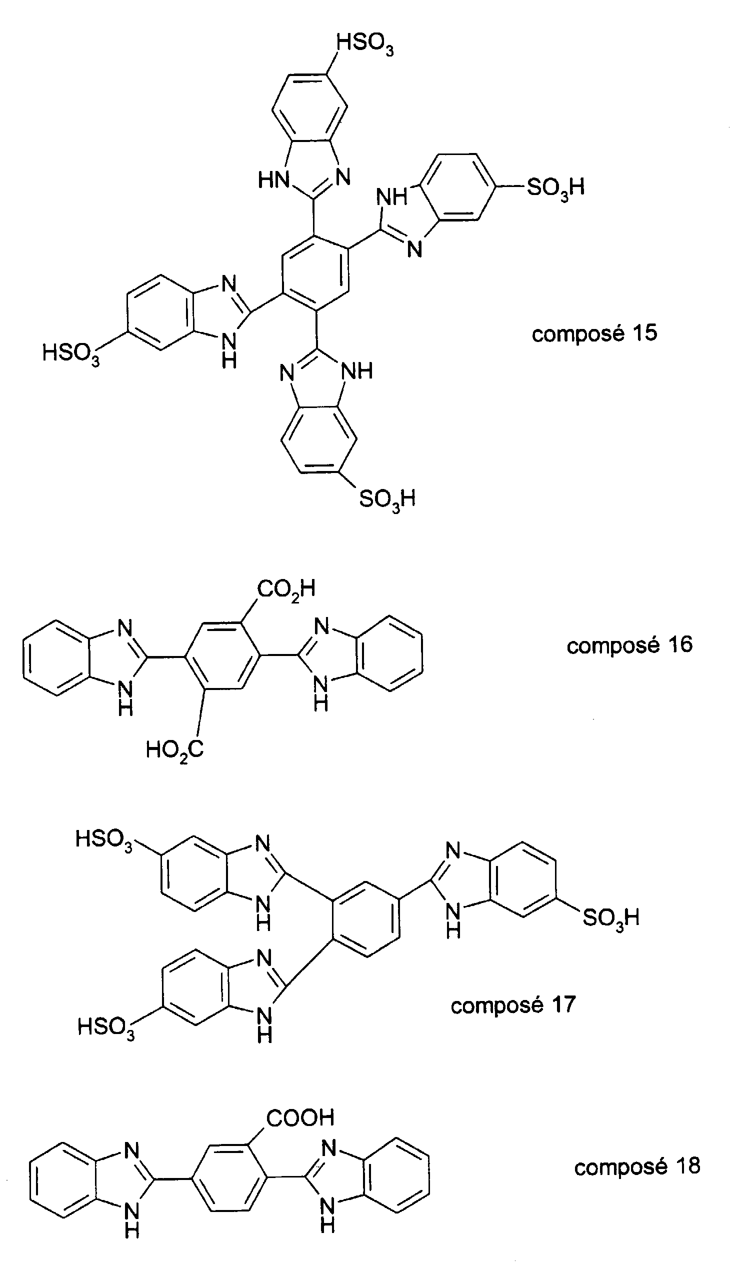

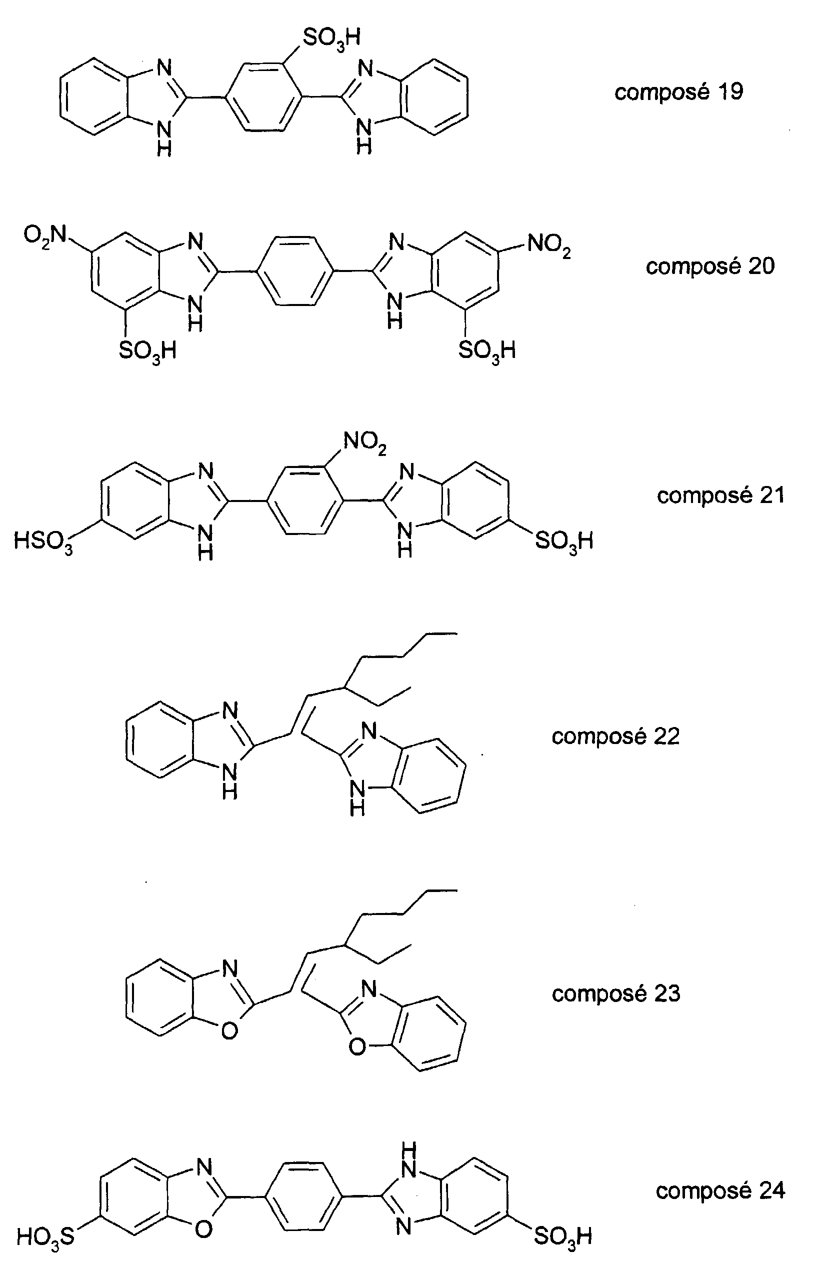

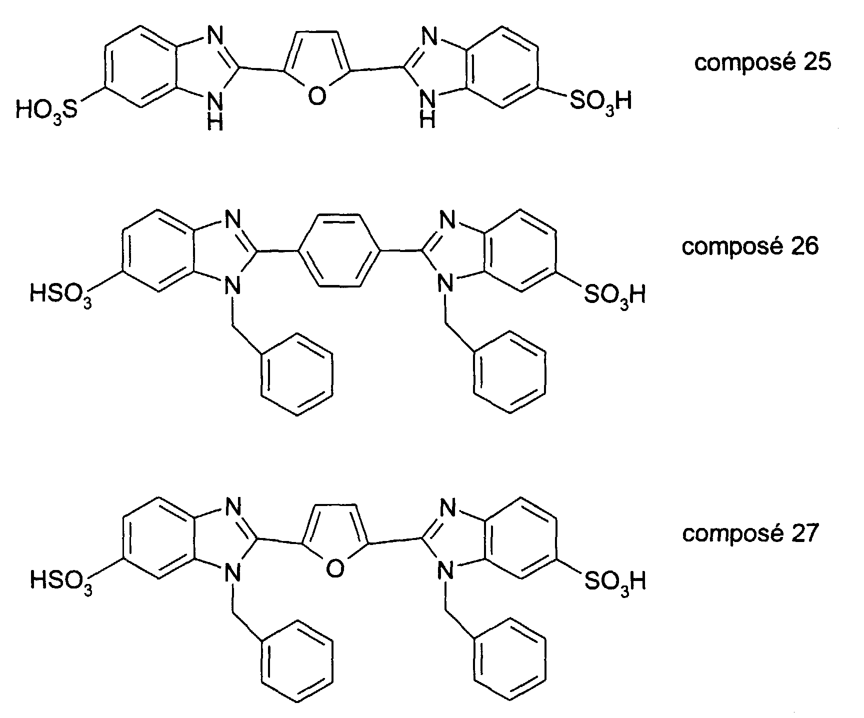

15. Composition selon l'une quelconque des revendications 11 à 14, où le composé de formule (III) est l'acide 1 ,4-bis-benzimidazolyl-phènylèn-3,3',5,5'-tétrasulfonique (composé 4) ou l'un des ses sels de stucture suivante:

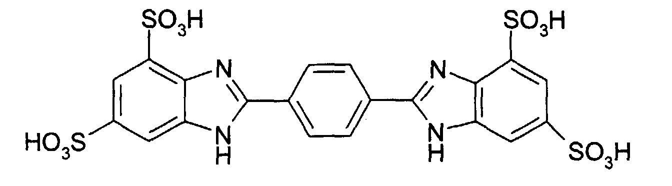

16. Composition selon l'une quelconque des revendications 11 à 13, caractérisée par le fait que le composé comportant au moins deux groupes benzoazolyle est choisi parmi les composés suivants ou l'un de leurs sels:

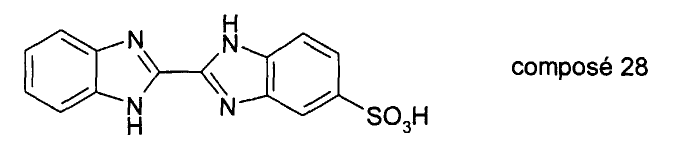

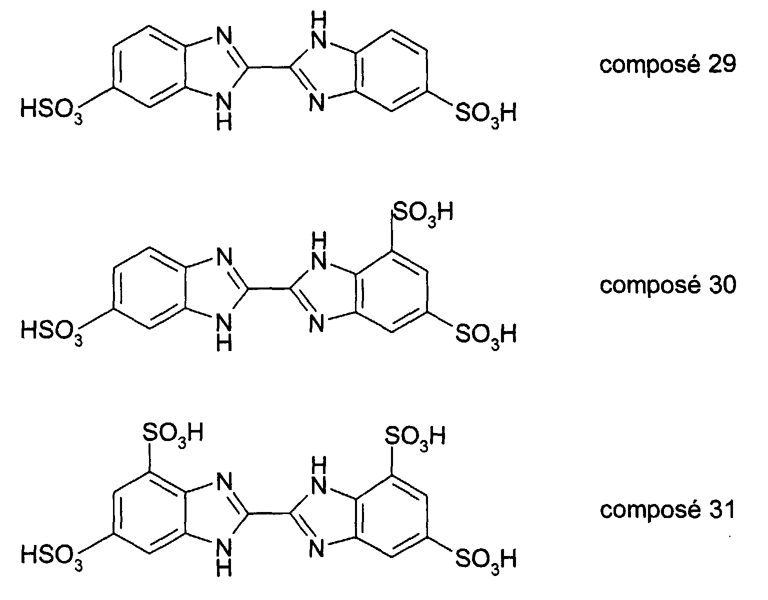

17. Composition selon l'une quelconque des revendications 1 à 10, caractérisée par le fait que le composé comportant au moins un groupe benzodiazolyle est choisi parmi les composés suivants ou l'un de leurs sels:

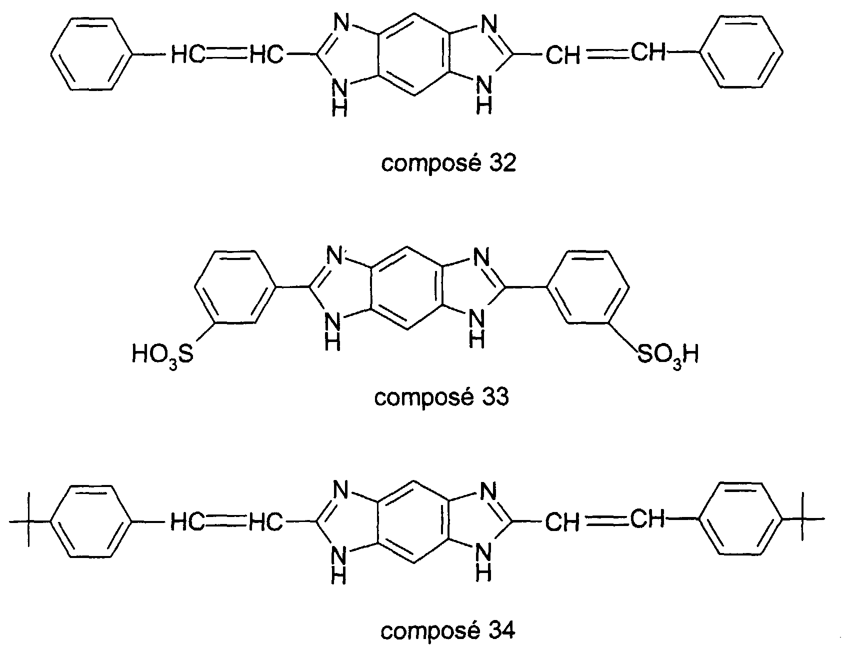

18. Composition selon l'une quelconque des revendications 1 à 17, caractérisée par le fait que le ou les composés à groupements benzoazolyle ou benzodiazolyle sont présents à une concentration comprise entre 0,1 et 15 % en poids par rapport au poids total de la composition.

19. Composition selon la revendication 18, caractérisée par le fait que ladite concentration est comprise entre 0,2 et 10% en poids par rapport au poids total de la composition.

20. Composition selon l'une quelconque des revendications 1 à 19, caractérisée par le fait que ledit support cosmétiquement acceptable se présente sous la forme d'une émulsion de type huile-dans-eau.

21. Composition selon l'une quelconque des revendications 1 à 20, caractérisée par le fait qu'elle comprend en outre un ou plusieurs filtres organiques complémentaires actifs dans l'UV-A et/ou UV-B, hydrophiles ou lipophiles, différents desdits premier et deuxième filtres.

22. Composition selon la revendication 21 caractérisée par le fait que lesdits filtres organiques complémentaires sont choisis parmi les dérivés cinnamiques, les dérivés salicyliques, les dérivés du camphre, les dérivés de triazine autres que ceux définis dans les revendications précédentes, les dérivés de benzimidazole autres que ceux définis dans les revendications précédentes, les dérivés de la benzophénone, les dérivés du dibenzoylméthane, les dérivés de β,β'-diphénylacrylate, les dérivés de l'acide p-aminobenzoïque, les polymères filtres et silicones filtres.

23. Composition selon l'une quelconque des revendications 1 à 22, caractérisée par le fait qu'elle comprend en outre, à titre d'agents photoprotecteurs UV complémentaires, des pigments ou des nanopigments d'oxydes métalliques, enrobés ou non.

24. Composition selon la revendication 23, caractérisée par le fait que lesdits pigments ou nanopigments sont choisis parmi les oxydes de titane, de zinc, de fer, de zirconium, de cérium et leurs mélanges, enrobés ou non.

25. Composition selon l'une quelconque des revendications 1 à 24, caractérisée par le fait qu'elle comprend en outre au moins un agent de bronzage et/ou de brunissage artificiel de la peau.

26. Composition selon l'une quelconque des revendications 1 à 25, caractérisée par le fait qu'elle comprend en outre au moins un adjuvant choisi parmi les corps gras, les solvants organiques, les épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants.

27. Composition selon l'une quelconque des revendications 1à 26, caractérisée par le fait qu'il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

28. Composition selon l'une quelconque des revendications 1 à 26, caractérisée par le fait qu'il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

29. Composition selon l'une quelconque des revendications 1 à 26, caractérisée par le fait qu'il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

30. Utilisation de la composition définie selon l'une quelconque des revendications 1 à 26 pour la fabrication de, compositions cosmétiques pour la protection de la peau et/ou des cheveux contre le rayonnement ultraviolet, en particulier le rayonnement solaire.

31. Procédé de traitement cosmétique pour protéger la peau et/ou les cheveux contre le rayonnement ultraviolet, en particulier le rayonnement solaire, caractérisé en ce qu'il consiste à appliquer sur ceux-ci une quantité efficace d'une composition telle que définie à l'une quelconque des revendications 1 à 29.
